(19) Europäisches Patentamt
European Patent Office
Office européen des brevets

(11) EP 1 510 510 A1

(12) # EUROPEAN PATENT APPLICATION

published in accordance with Art. 158(3) EPC

(43) Date of publication:
**02.03.2005 Bulletin 2005/09**

(21) Application number: **03756115.6**

(22) Date of filing: **27.05.2003**

(51) Int Cl.[7]: **C07B 37/04**, C07C 51/367, C07C 65/105, C07C 51/353, C07C 63/04, C07C 29/40, C07C 33/26, C07C 231/12, C07C 235/42, C07C 37/20, C07C 39/12, C07D 491/107, C07D 307/88, C07D 307/89, C07D 307/94

(86) International application number:
**PCT/JP2003/006624**

(87) International publication number:
**WO 2003/101916 (11.12.2003 Gazette 2003/50)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **31.05.2002 JP 2002160759**

(71) Applicant: **BANYU PHARMACEUTICAL CO., LTD.**
**Chuo-ku, Tokyo 103-8416 (JP)**

(72) Inventors:
- **KATO, Shinji**
  **Nagoya-shi, Aichi 456-0058 (JP)**
- **NONOYAMA, Nobuaki,**
  **Banyu Pharmaceutical Co., Ltd.**
  **Okazaki-shi, Aichi 444-0858 (JP)**
- **MASE, Toshiaki**
  **Okazaki-shi, Aichi 444-0822 (JP)**

(74) Representative: **Teipel, Stephan, Dr. et al**
**Lederer & Keller**
**Patentanwälte**
**Prinzregentenstrasse 16**
**80538 München (DE)**

(54) **METHOD OF SUBSTITUENT INTRODUCTION THROUGH HALOGEN-METAL EXCHANGE REACTION**

(57) A method of exchanging a halogen atom of a halide, which has a group containing an acidic proton and in which one or more halogen atom(s) is/are substituted on a carbon atom of the carbon-carbon double bond, with a metal atom by halogen-metal-exchange reaction and introducing an electrophilic reagent into the carbon atom to which the metal atom is attached. The above method is an industrially excellent method of introducing a substituent by halogen-metal exchange reaction.

EP 1 510 510 A1

**Description**

TECHNICAL FIELD

The present invention is useful in the field of fine chemicals such as pharmaceuticals, agricultural chemicals, perfumes and the like. In more detail, the present invention relates to a method of introducing a substituent to a production intermediate for a compound which is useful in the field of pharmaceuticals and the like, and a novel production method which is advantageous for the production.

BACKGROUND ART

Some of the compounds produced by a method of introducing a substituent by halogen-metal exchange reaction according to the present invention are useful as a production intermediate for the compound which is useful as a neuropeptide Y receptor antagonist disclosed, for example, in WO 01/14376.
A method of introducing a substituent by halogen-metal exchange reaction has often been utilized in the production of intermediates for pharmaceuticals, including the aforementioned compounds.
Known methods of introducing a substituent by halogen-metal exchange reaction are disclosed in, for example, ACCOUNTS OF CHEMICAL RESEARCH, vol.15, p. 300-305, 1982, William E. parham et al., JOURNAL OF ORGANIC CHEMISTRY, vol.39, p.2051-2053, 1974, William E. parham et al. and JOURNAL OF ORGANIC CHEMISTRY, vol.41, p.2628-2633, 1976, WilliamE. Parham et al., however, those production methods disclosed in said literatures are carried out under an extreme cold condition (-78 °C) . In the industrial production, setting such an extreme cold condition requires a particular cooling system, thus said production methods are inefficient and not desirable for the industrial production.
Recently, a report by Kazuya Kitagawa et al. in ANGEWANTE CHEMIE INTERNATIONAL EDITION, vol. 39, p.2481-2483, and WO 01/57046 also disclosed a method of introducing a substituent by halogen-metal exchange reaction in which an extreme cold condition was not necessary. This method of introducing a substituent by halogen-metal exchange reaction, however, has a limitation that the compound having a halogen atom, which is a starting material, is not applicable when it includes a substituent containing an acidic proton. Therefore, in case where a starting material includes a substituent containing an acidic proton, it is necessary to protect the substituent and then to remove the protecting group after the reaction, resulting in additional processes, complex process control and reduced yield. Accordingly, said method is inefficient for the purpose of industrial production.

DISCLOSURE OF THE INVENTION

An object of the present invention is to improve problems relating to the known methods of introducing a substituent by halogen-metal-exchange reaction and to provide an industrially superior method of introducing a substituent by halogen-metal exchange reaction.
The present inventors conducted intensive studies on a method of introducing a substituent by halogen-metal exchange reaction. As a result, they found that a halide which has a substituent containing an acidic proton can be employed as a starting material without protection of the substituent, and the condition of extreme low temperature (-78 °C) can be avoided when a halide, which has a substituent containing an acidic proton and in which the carbon atom(s) of the carbon-carbon double bond is/are substituted by one or more halogen(s), as a starting material is reacted with a magnesium halide compound, a magnesium compound, or a mixture thereof, and subsequently reacted with a lithium compound and then with an electrophilic reagent. The present inventors carried out further studies on the basis of those findings, and finally completed the present invention.
Namely, the present invention relates to:

(1) A method of exchanging a halogen atom of a halide with a metal atom by halogen-metal exchange reaction and introducing an electrophilic reagent into a carbon atom to which the metal atom is attached, which comprises reacting a halide which has a group containing an acidic proton and in which one or more halogen atom(s) is/are substituted on a carbon atom of the carbon-carbon double bond, with a magnesium halide represented by the formula (I):

[I]

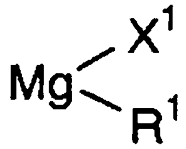

(wherein $R^1$ is a hydrocarbon group, and $X^1$ is a halogen atom), or a magnesium compound represented by the formula (II):

[II]

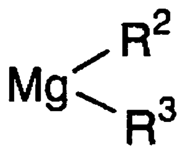

(wherein $R^2$ and $R^3$ are each independently a hydrocarbon group), or a mixture thereof, followed by successive reactions with a lithium compound represented by the formula (III):

[III]

**Li -$R^4$**

(wherein $R^4$ is a hydrocarbon group), and then with an electrophilic reagent, and then optionally treating the resulting product with an acid,
(2) The method according to the above (1), wherein the hydrocarbon group is an alkyl group, an aromatic group or an aralkyl group,
(3) The method according to the above (1) or (2), wherein the halide which has a group containing an acidic proton and in which one or more halogen atom(s) is/are substituted with the carbon atom of the carbon-carbon double bond is a compound represented by the formula (IV):

[IV]

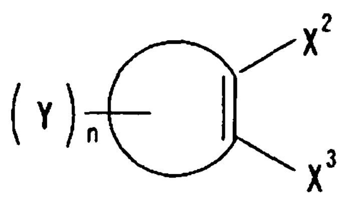

wherein

$X^2$ is a halogen atom;
$X^3$ is a hydrogen atom or a halogen atom;
n is an integer of 1 to 4;
Y is a carboxyl group, a hydroxymethyl group, a hydroxy group, a sulfamoyl group, a group of the formula: $-SO_3H$, a phosphono group, a group of the formula: $-NH-Y^1$ (wherein $Y^1$ is a hydrogen atom, a hydroxy group, an alkyl group, a carboxyl group, an aromatic group, a benzyloxycarbonyl group, an alkoxycarbonyl group or a phenyloxycarbonyl group), a group of the formula: $-CO-NH-Y^2$ ($Y^2$ is a hydrogen atom, an alkyl group or an aromatic group) or a group of the formula: $-NH-SO_2-Y^3$ (wherein $Y^3$ is a hydrogen atom, an alkyl group or an aromatic group), and
the group represented by the formula:

[V]

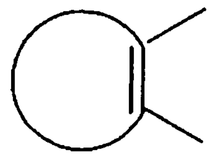

is a divalent homocyclic or heterocyclic group, and when n is more than 1, each Y may be the same or different,

(4) The method according to the above (2) or (3) , wherein the alkyl group is a linear or branched $C_1$-$C_6$ alkyl group, and the aromatic group is phenyl, 1-naphthyl, 2-naphthyl or benzyl,

(5) The method according to any one of the above (1) to (4), wherein the homocyclic or heterocyclic ring is a benzene ring, a naphthalene ring, an indene ring, an indane ring, a pyridine ring, a pyridazine ring, a pyrimidine ring, a pyrazine ring, a thiophen ring, a furan ring, a pyran ring, an isobenzofuran ring, a pyrrole ring, an imidazole ring, a pyrazole ring, an isothiazole ring, a thiazole ring, an oxazole ring, an isoxazole ring, a quinoline ring, an isoquinoline ring, a benzofuran ring, an indole ring, an isoindole ring, a phthalazine ring, a quinoxaline ring, a benzimidazole ring, a 1,8-naphthyridine ring, a benzoxazole ring, a benzothiazole ring, a benzothiophen ring, a cinnoline ring or a quinazoline ring,

(6) The method according to any one of the above (1) to (5), wherein the magnesium halide represented by the formula (I) is methyl magnesium bromide, methyl magnesium chloride, ethyl magnesium chloride, n-propyl magnesium chloride, isopropyl magnesium chloride, isopropyl magnesium bromide, n-butyl magnesium chloride, n-butyl magnesium bromide, isobutyl magnesium chloride, phenyl magnesium chloride or phenyl magnesium bromide, and the magnesium compound represented by the formula (II) is dimethylmagnesium, diethylmagnesium, di-n-propylmagnesium, diisopropylmagnesium, di-n-butylmagnesium, diisobutylmagnesium, di-sec-butylmagnesium, n-butyl(sec-butyl)magnesium or diphenylmagnesium,

(7) The method according to any one of the above (1) to (6), wherein the lithium compound represented by the formula (III) is methyllithium, n-butyllithium, sec-butyllithium, tert-butyllithium or phenyllithium,

(8) The method according to any one of the above (1) to (7), wherein the electrophilic reagent is benzaldehyde, methyl iodide, N,N-dimethylformamide, 1-benzyl-4-piperidone, 1-tert-butoxycarbonyl-4-piperidone, allyl bromide, cyclohexanone, n-heptanal, n-hexanal, acetone or methyl ethyl ketone,

(9) The method according to the above (1) , which comprises reacting 2-bromobenzoic acid represented by the formula:

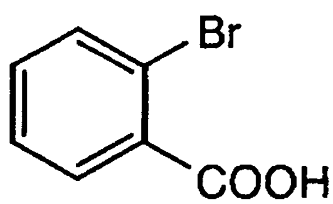

with di-n-butylmagnesium, di-sec-butylmagnesium, n-butyl(sec-butyl)magnesium, isopropyl magnesium chloride, n-butyl magnesium bromide, or a mixture thereof, followed by successive reactions with n-butyllithium, and then with a piperidone derivative represented by the formula:

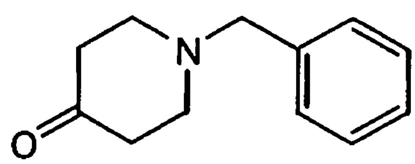

, and then treating the product with an acid to give a spiro compound represented by the formula:

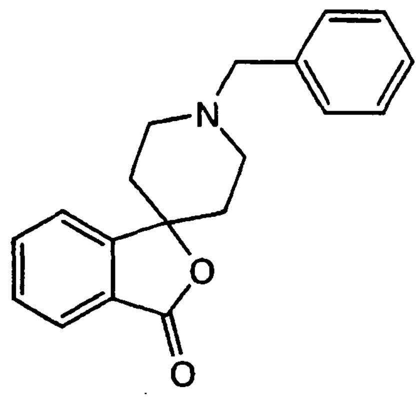

;

and

(10) A method of the preparation of the spiro compound described in the above (9), which comprises using the method according to the above (9).

BEST MODE FOR CARRYING OUT THE INVENTION

The present invention will be described specifically and in detail below.

The "halogen atom" includes, for example, a chlorine atom, a bromine atom, a fluorine atom and an iodine atom, among which a chlorine atom, a bromine atom or an iodine atom is preferable.

The "hydrocarbon group" includes, for example, $C_1$-$C_6$ alkyl group, $C_6$-$C_{14}$ aromatic group and aralkyl group consisting of said aromatic group and alkyl group, among which $C_1$-$C_6$ alkyl group, aromatic group such as phenyl, naphthyl and the like, or aralkyl group such as benzyl and the like is preferable.

The "alkyl group" preferably includes $C_1$-$C_6$ alkyl group, for example, straight or branched $C_1$-$C_6$ alkyl group such as methyl, ethyl, n-propyl, isopropyl, n-butyl, isobutyl, tert-butyl, sec-butyl, n-pentyl, isopentyl, neopentyl, n-hexyl, isohexyl and the like, among which methyl, ethyl, n-propyl, n-butyl or n-hexyl is more preferable.

The "alkoxycarbonyl group" preferably includes $C_2$-$C_7$ alkoxycarbonyl group, for example, methoxycarbonyl, ethoxycarbonyl, n-propoxycarbonyl, isopropoxycarbonyl, n-butoxycarbonyl, isobutoxycarbonyl, sec-butoxycarbonyl, tert-butoxycarbonyl, n-pentyloxycarbonyl, n-hexyloxycarbonyl and the like, among which methoxycarbonyl or tert-butoxycarbonyl is preferable.

The "halide which has a group containing an acidic proton and in which one or more halogen atom(s) is/are substituted on the carbon atom of the carbon-carbon double bond" includes the compound in which one of the carbons of the double bond is substituted with a halogen atom, or the compound in which both of the carbons of the double bond are substituted with a halogen atom, preferably the compound represented by the formula (IV) mentioned above.

The "group containing an acidic proton" preferably includes a group represented by the above Y.

As for $X^3$, the case where $X^3$ is a hydrogen atom is more preferable than the case where $X^3$ is a halogen atom.

The homocycle preferably includes $C_6$-$C_{20}$ ring such as benzene ring, naphthalene ring, etc., and the heterocycle preferably includes 5- to 10-membered aromatic heterocycle containing, in addition to carbon atoms, one to four atom(s) independently selected from the group consisting of nitrogen, sulfur and oxygen as atoms forming the ring. Such homocycle and heterocycle may be further substituted by a common group, for example, the above-mentioned halogen atom, preferably $C_1$-$C_6$ alkyl group, dialkylamino group such as dimethylamino, diethylamino, etc., preferably $C_1$-$C_4$ alkenyl group such as vinyl, allyl, etc., preferably $C_1$-$C_6$ alkoxy group such as methoxy, ethoxy, n-propyloxy, n-butoxy, etc. , preferably $C_1$-$C_4$ alkylthio group such as methylthio, ethylthio, n-propylthio, n-butylthio, etc. , preferably $C_2$-$C_7$ alkoxycarbonyl group, benzyloxycarbonyl group and the like.

The electrophilic reagent may be any kind of molecules containing a functional group capable of accepting an electron, and preferably includes compounds reacting with a group having high electron density or with lone pair. Since the exchange reaction of the present invention is an improved halogen-metal exchange reaction with Grignard reagents and/or organolithium reagents, the aforementioned compounds include any electrophilic reagents employed for the halogen-metal exchange reaction, such as Grignard reagents and/or organolithium reagents.

Typical examples of the compounds having an electrophilic group include halogen (e.g. chlorine, bromine or iodine), carbon dioxide, solid sulfur, sulfur dioxide, oxygen, benzaldehyde, methyl iodide, N,N-dimethylformamide, 1-benzyl-4-piperidone, 1-tert-butoxycarbonyl-4-piperidone, allyl bromide, cyclohexanone, n-heptanal, n-hexanal, acetone, methylethylketone and the like.

The preferable scope of the electrophilic reagent is also described by the compound of formulae (1) to (21) given below.

(1) A compound represented by the formula:

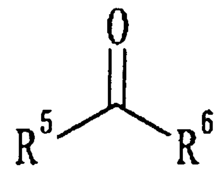

(1)

(wherein $R^5$ and $R^6$ are each independently hydrogen, preferably $C_1$-$C_4$ alkyl, a halogen atom, preferably $C_1$-$C_4$ alkoxy, preferably $C_1$-$C_4$ alkylthio, preferably $C_6$-$C_{10}$ arylthio, N,N-dialkylamino (wherein the alkyl moiety preferably having 1 to 4 carbon atoms) , preferably $C_5$-$C_{12}$ aryl or preferably $C_4$-$C_{10}$ heterocycle (optionally containing, as a hetero atom, one to four members selected from the group consisting of oxygen, a group of the formula: S(→O) $n^1$ ($n^1$ is 0, 1 or 2), a group of the formula: N→O, a group of the formula: =N- and a group of the formula: $NR^7$ ($R^7$ is $C_1$-$C_4$ alkyl or benzyl)).

Typical examples of the compounds (1) used include ethyl formate, N,N-dimethylaminocarbonyl chloride, N, N-dimethylacetamide, acetyl chloride, ethyl benzoate, diethyl carbonate, ethyl chlorocarbonate, ethyl chlorothiocarbonate and the like.

(2) An acid anhydride represented by the formula:

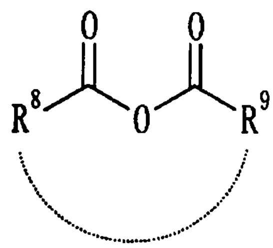

(2)

(wherein $R^8$ and $R^9$ are each independently preferably $C_1$-$C_4$ alkyl, preferably $C_5$-$C_{12}$ aryl, preferably $C_7$-$C_{13}$ aralkyl or preferably $C_4$-$C_{10}$ heterocycle (optionally containing, as a hetero atom, one to four members selected from the group consisting of oxygen, a group of the formula: S(→O)$n^2$ ($n^2$ is 0, 1 or 2), a group of the formula: N→O, a group of the formula: =N- and a group of the formula: $NR^{10}$ ($R^{10}$ is preferably $C_1$-$C_4$ alkyl or benzyl) ) , or $R^8$ and $R^9$, taken together, may form preferably $C_2$-$C_4$ alkylene or phenylene).

Typical examples of the compound (2) used include acetic anhydride, succinic anhydride, propionic anhydride, benzoic anhydride, maleic anhydride and the like.

(3) A compound represented by the formula:

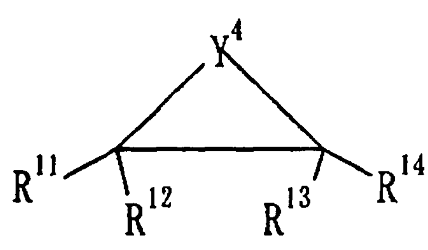

(3)

(wherein $R^{11}$, $R^{12}$, $R^{13}$ and $R^{14}$ are each independently a hydrogen atom, preferably $C_1$-$C_4$ alkyl or phenyl, or either of $R^{11}$ and $R^{12}$ and either of $R^{13}$ and $R^{14}$, taken together, may form preferably $C_3$-$C_{12}$ alkylene, and $Y^4$ is an oxygen atom or a group of the formula: N-$R^{15}$ ($R^{15}$ is preferably $C_1$-$C_4$ alkyl, preferably $C_1$-$C_4$ alkylsulfonyl, preferably $C_1$-$C_5$ alkanoyl, preferably $C_1$-$C_5$ alkoxycarbonyl or a group of the formula: S(→O)$n^3$ ($n^3$ is 0, 1 or 2)).

Typical examples of the compound (3) used include aziridines and ethylene oxide derivatives, such as ethylene oxide, 2-methyl-1,2-epoxypropane, 7-thiabicyclo[4,1,0]heptane, 6-thiabicyclo[3,1,0]hexane, 7-oxabicyclo[4,1,0]heptane, 6-oxabicyclo[3,1,0]hexane, 7-azabicyclo[4,1,0]heptane, 6-azabicyclo[3,1,0]hexane, 7-ethoxycarbonyl-7-azabicyclo[4,1,0]heptane, 6-ethoxycarbonyl-6-azabicyclo[3,1,0]hexane and the like.

(4) An $\alpha,\beta$-unsaturated carbonyl compound represented by the formula:

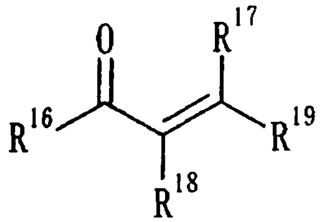

(4)

(wherein $R^{16}$, $R^{17}$, $R^{18}$ and $R^{19}$ are each independently a hydrogen atom, preferably $C_1$-$C_4$ alkyl or phenyl).

Typical examples of the compound (4) used include $\alpha,\beta$-unsaturated ketone derivatives such as 3-oxo-1,3-diphenyl-1-propene, 2-methyl-3-oxo-3-diphenyl-1-propene and the like.

(5) A halogenated ethane represented by the formula:

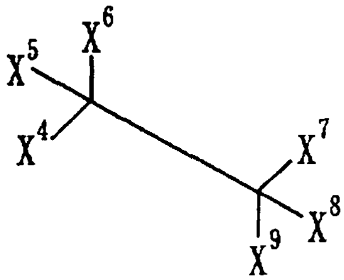

(5)

(wherein $X^4$ , $X^5$, $X^6$, $X^7$, $X^8$ and $X^9$ are each independently a hydrogen atom or a halogen atom, provided that at least one of $X^4$, $X^5$ and $X^6$ is a halogen atom, and at least one of $X^7$, $X^8$ and $X^9$ is a halogen atom).

Typical examples of the compound (5) used include halogenated ethanes such as hexachloroethane, hexafluoroethane, 1,1,2,2-tetrabromoethane, 1,2-dibromoethane, 1,2-diiodoethane, pentafluoroiodoethane, 1,2-dibromo-1,1,2,2-tetrafluoroethane, 1,2-dichloro-1,1,2,2-tetrafluroethane, 1,1,1-trichloro-2,2,2-trifluoroethane, 1,2,2-trifluoroethane, 2,2,1-trichloroethane and the like.

(6) A compound represented by the formula:

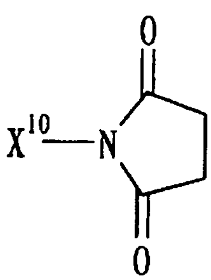

(6)

(wherein $X^{10}$ is a halogen atom).

Typical examples of the compounds (6) used include succinimides such as N-iodosuccinimide, N-bromosuccinimide, N-chlorosuccinimide and the like.

(7) A compound represented by the formula:

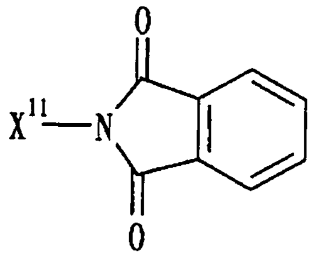

(7)

(wherein $X^{11}$ is a halogen atom).

Typical examples of the compounds (7) used include phthalimides such as N-bromophthalimide, N-chlorophthalimide and the like.

(8) A compound represented by the formula:

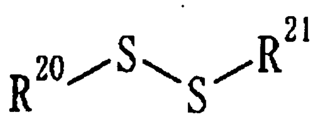

(8)

(wherein $R^{20}$ and $R^{21}$ are each independently preferably $C_1$-$C_4$ alkyl or phenyl).

Typical examples of the compounds (8) used include disulfide derivatives such as dimethyl disulfide, diethyl disulfide, di-n-propyl disulfide, di-n-butyl disulfide, diphenyl disulfide and the like.

(9) A compound represented by the formula:

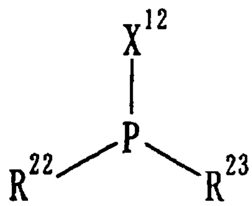

(9)

(wherein $X^{12}$ is a halogen atom, and $R^{22}$ and $R^{23}$ are each independently preferably $C_1$-$C_4$ alkyl or phenyl).

Typical examples of the compounds (9) used include phosphine derivatives such as chlorodiphenylphosphine, chlorodimethylphosphine, bromodiphenylphosphine, bromodimethylphosphine and the like.

(10) A compound represented by the formula:

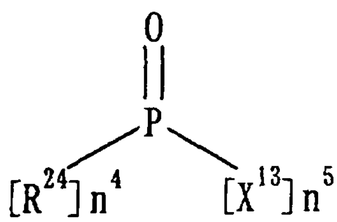

(10)

(wherein $X^{13}$ is a halogen atom or phenyl, $R^{24}$ is preferably $C_1$-$C_4$ alkyl, phenyl, preferably $C_1$-$C_4$ alkoxy, dialkylamino (wherein the alkyl moiety preferably having 1 to 4 carbon atoms) or preferably $C_2$-$C_5$ alkoxycarbonylamino, $n^4$ is 0, 1 or 2, and $n^5$ is 1, 2 or 3).

Typical examples of the compounds (10) used include dimethyl chlorophosphonate, triphenylphosphine oxide, diphenyl chlorophosphonate and the like.

(11) A compound represented by the formula:

$$R^{25}—C\equiv N \qquad (II)$$

(wherein $R^{25}$ is preferably $C_1$-$C_4$ alkyl or phenyl).

Typical examples of the compounds (11) used include acetonitrile, propionitrile, butyronitrile, benzonitrile and the like.

(12) A compound represented by the formula:

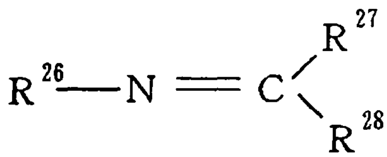

(12)

(wherein $R^{26}$ is preferably $C_1$-$C_4$ alkyl, dialkylamino (wherein the alkyl moiety preferably having 1 to 4 carbon atoms), hydroxy or $C_1$-$C_4$ alkoxy, and $R^{27}$ and $R^{28}$ are each independently preferably $C_1$-$C_4$ alkyl or phenyl).

Typical examples of the compounds (12) used include benzophenoneimine, acetone-oxime and the like.

(13) A compound represented by the formula:

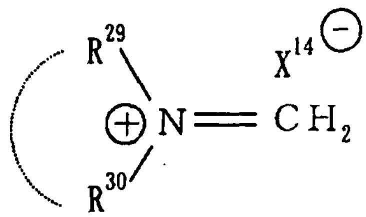

(13)

(wherein $R^{29}$ and $R^{30}$ are each independently preferably $C_1$-$C_4$ alkyl or phenyl, or $R^{29}$ and $R^{30}$, taken together, may form ethylene or propylene, and $X^{16}$ is a halogen atom).

Typical examples of the compounds (13) used include compounds represented by the following formulae:

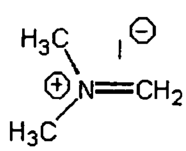 , 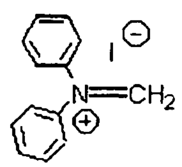 , 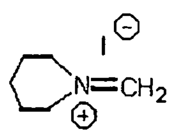 , 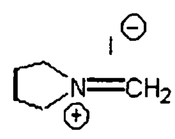 , 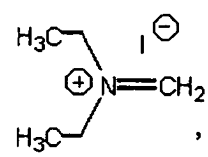 ,

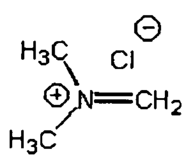 , 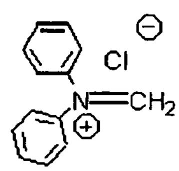 , 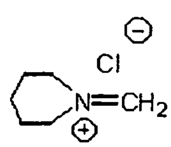 , 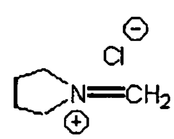 , 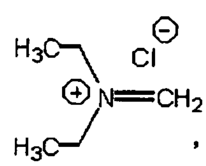 ,

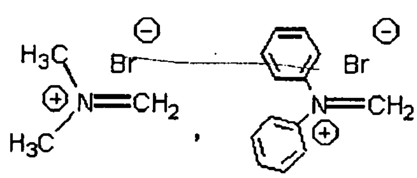 , 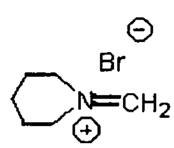 , 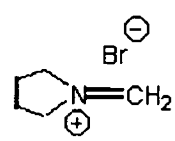 and 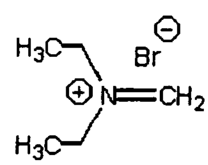

(14) A compound represented by the formula:

$$X^{15} — M^{1} \quad (14)$$

(wherein $X^{15}$ is a halogen atom or preferably $C_1$-$C_4$ alkoxy and, $M^1$ is a copper atom, a silver atom or a thallium atom).

Typical examples of the compounds (14) used include monovalent metal compounds such as cuprous iodide, silver iodide, thallium chloride, thallium bromide, cuprous chloride, silver chloride, cuprous bromide, silver bromide and the like.

(15) A compound represented by the formula:

$$X^{16} — M^{2} — X^{16} \quad (15)$$

(wherein $X^{16}$ is a halogen atom or preferably $C_1$-$C_4$ alkoxy, $M^2$ is a zinc atom, a mercury atom, a nickel atom, a manganese atom, a palladium atom, an iron atom or a copper atom).

Typical examples of the compounds (15) used include divalent metal compounds such as ferrous chloride, zinc chloride, mercuric chloride, cupric chloride, nickel chloride, palladium chloride, manganese chloride, manganese bromide, manganese iodide, manganese fluoride and the like.

(16) A compound represented by the formula:

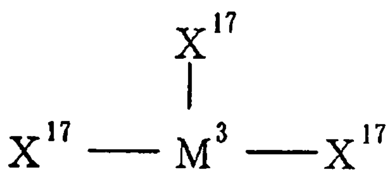

(16)

(wherein $X^{17}$ is a halogen atom or preferably $C_1$-$C_4$ alkoxy, $M^3$ is an aluminum atom, a titanium atom, a gallium atom, an indium atom, a vanadium atom, a ruthenium atom, a cobalt atom, a rare earth atom, a scandium atom, an yttrium atom or a hafnium atom) .

Typical examples of the compound (16) used include trivalent metal compounds such as zirconium chloride, ferric chloride, ruthenium chloride, ruthenium bromide, ruthenium iodide, cobaltic chloride, scandium chloride, yttrium chloride, lanthanum chloride, ytterbium chloride, titanium chloride, aluminum chloride, vanadium chloride, vanadium iodide, vanadium fluoride, vanadium bromide, gallium chloride and the like.

(17) A compound represented by the formula:

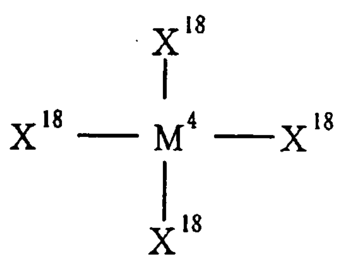

(17)

(wherein $X^{18}$ is a halogen atom or preferably $C_1$-$C_4$ alkoxy, $M^4$ is an osmium atom, a germanium atom, a titanium atom, a zirconium atom, a vanadium atom, a manganese atom, a ruthenium atom or a tin atom).

Typical examples of the compounds (17) used include tetravalent metal compounds such as germanium chloride, germanium bromide, germanium fluoride, germanium iodide, germanium methoxide, germanium ethoxide, germanium isopropoxide, titanium chloride, titanium bromide, titanium iodide, titanium fluoride, titanium methoxide, titanium ethoxide, titanium isopropoxide, titanium butoxide, zirconium chloride, zirconium bromide, zirconium fluoride, zirconium iodide, zirconium methoxide, zirconium ethoxide, zirconium isopropoxide, zirconium butoxide, zirconium tert-butoxide, vanadium chloride, vanadium bromide, vanadium fluoride, vanadium iodide, tin chloride,

tin bromide, tin fluoride, tin iodide and the like.

(18) A compound represented by the formula:

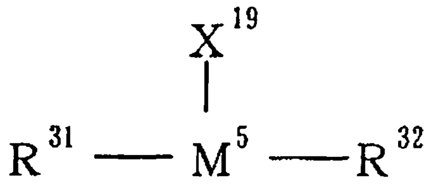

(18)

(wherein $R^{31}$ and $R^{32}$ are each independently preferably $C_1$-$C_4$ alkyl, $X^{19}$ is a halogen atom or preferably $C_1$-$C_4$ alkoxy, and $M^5$ is an aluminum atom).

Typical examples of the compounds (18) used include aluminum (III) compounds such as diethylaluminum chloride, diethylaluminum bromide, diethylaluminum ethoxide, diethylaluminum iodide, diisopropylaluminum chloride, dimethylaluminum iodide, dimethylaluminum chloride, dimethylaluminum bromide, dimethylaluminum iodide and the like.

(19) A compound represented by the formula:

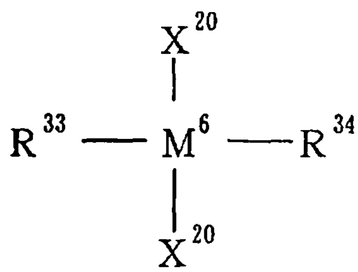

(19)

(wherein $R^{33}$ and $R^{34}$ are each independently preferably $C_1$-$C_4$ alkyl or phenyl, $X^{20}$ is a halogen atom or preferably $C_1$-$C_4$ alkoxy, and $M^6$ is a tin atom).

Typical examples of the compounds (19) used include tin (IV) compounds such as di-n-butyltin dichloride, di-n-butyltin dibromide, di-n-butyltin dimethoxide, dimethyltin dibromide, diphenyltin dibromide, dimethyltin dichloride, diphenyltin dichloride and the like.

(20) A compound represented by the formula:

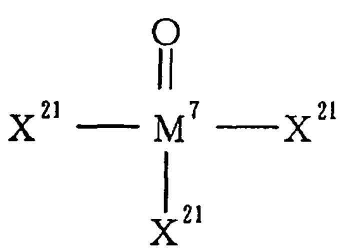

(20)

(wherein $X^{21}$ is a halogen atom or preferably $C_1$-$C_4$ alkoxy, and $M^7$ is a vanadium atom).

Typical examples of the compounds (20) used include vanadium (V) compounds such as vanadium oxytrichloride, vanadium oxytrifluoride, vanadium oxytribromide, vanadium oxytriethoxide, vanadium oxytriisopropoxide, vanadium oxytri-n-butoxide, vanadium oxytri-tert-butoxide and the like.

(21) A compound represented by the formula:

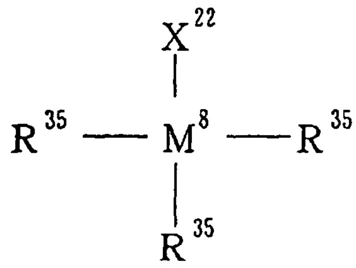

(21)

(wherein $X^{22}$ is trifluoromethanesulfonyloxy or a halogen atom, $R^{35}$ is preferably $C_1$-$C_4$ alkyl or phenyl, and $M^8$ is silicon atom)

Typical examples of the compounds (21) used include silane derivatives such as chlorotrimethylsilane, chlorotriethylsilane, chlorotri-n-butylsilane, chlorotriisoproylsilane, chlorotriphenylsilane, bromotrimethylsilane, bromotriethylsilane, bromotri-n-butylsilane, bromotriisopropylsilane, bromotriphenylsilane, trifluoromethanesulfonyloxytrimethylsilane, trifluoromethanesulfonyloxytriethylsilane, trifluoromethanesulfonyloxytri-n-butylsilane, trifluoromethanesulfonyloxytriisopropylsilane, iodotrimethylsilane and the like.

Among aforementioned compounds (1) to (21), benzaldehyde, methyl iodide, N,N-dimethylformamide, 1-benzyl-4-piperidone, 1-tert-butoxycarbonyl-4-piperidone, allyl bromide, cyclohexanone, n-heptanal, n-hexanal, acetone or methyl ethyl ketone is preferable as a compound having an electrophilic group.

The definitions of symbols in the formulae (1) to (21) are as follows:

The "$C_5$-$C_{12}$ aryl" includes, for example, cyclopentadienyl, phenyl, indenyl, biphenylyl, naphthyl and the like, among which phenyl is preferable.

The "$C_1$-$C_{12}$ alkyl" includes, for example, a linear or branched alkyl group such as methyl, ethyl, n-propyl, isopropyl, n-butyl, isobutyl, tert-butyl, n-pentyl, n-hexyl, n-heptyl, n-octyl, n-nonyl, n-decyl, n-undecyl, n-dodecyl and the like, among which methyl, ethyl, n-propyl, isopropyl, n-butyl, n-pentyl, n-hexyl, n-decyl, n-dodecyl or the like is preferable, and methyl, ethyl, n-propyl, n-butyl, n-pentyl, n-hexyl or n-dodecyl is more preferable.

The "$C_1$-$C_4$ alkyl" includes, for example, a linear or branched alkyl group such as methyl, ethyl, n-propyl, isopropyl, n-butyl, isobutyl, tert-butyl and the like, among which methyl, ethyl, n-propyl, isopropyl, n-butyl or the like is preferable, and methyl, ethyl, n-propyl or n-butyl is more preferable.

The "$C_2$-$C_4$ alkenyl" includes, for example, a linear alkenyl group such as vinyl, allyl, 1-propenyl, 1-butenyl, 2-butenyl and the like, among which vinyl or allyl is preferable.

The "$C_6$-$C_{10}$ aryl" includes, for example, an aryl group such as phenyl, o-tolyl, m-tolyl, p-tolyl, o-anisyl, m-anisyl, p-anisyl, 1-naphthyl, 2-naphthyl and the like, among which phenyl, p-tolyl, p-anisyl or 2-naphthyl is preferable.

The "$C_6$-$C_{10}$ arylthio" includes, for example, an arylthio group such as phenylthio, o-tolylthio, m-tolylthio, p-tolylthio, o-anisylthio, m-anisylthio, p-anisylthio, 1-naphthylthio, 2-naphthylthio and the like, among which phenylthio, p-tolylthio, p-anisylthio or 2-naphthylthio is preferable.

The "$C_7$-$C_{13}$ aralkyl" includes, for example, an aralkyl group such as benzyl, o-tolylmethyl, m-tolylmethyl, p-tolylmethyl, o-anisylmethyl, m-anisylmethyl, p-anisylmethyl, benzhydryl, 1-naphthylmethyl, 2-naphthylmethyl and the like, among which benzyl, p-tolylmethyl, p-anisylmethyl or 2-naphthylmethyl is preferable.

The "$C_4$-$C_8$ heteroaryl" includes, for example, a heteroaryl group such as 2-thienyl, 3-thienyl, 2-furyl, 3-furyl, benzofuranyl, benzothienyl and the like, among which 2-thienyl, 3-thienyl, 2-furyl or 3-furyl is preferable.

The "$C_1$-$C_4$ alkoxy" includes, for example, a linear or branched alkoxy group such as methoxy, ethoxy, n-propoxy, isopropoxy, n-butoxy, isobutoxy, tert-butoxy and the like, among which methoxy, ethoxy, n-propoxy or n-butoxy is preferable, and methoxy, ethoxy or n-butoxy is more preferable.

The "$C_2$-$C_5$ alkoxycarbonylamino" includes, for example, a linear or branched alkoxycarbonylamino group such as methoxcycarbonylamino, ethoxycarbonylamino, n-propoxycarbonylamino, isopropoxycarbonylamino, n-butoxycarbonylamino, isobutoxycarbonylamino, tert-butoxycarbonylamino and the like, among which methoxcycarbonylamino, ethoxycarbonylamino, n-propoxycarbonylamino, n-butoxycarbonylamino or the like is preferable, and methoxcycarbonylamino, ethoxycarbonylamino or n-butoxycarbonylamino is more preferable.

The "$C_1$-$C_4$ alkylthio" includes, for example, a linear or branched alkylthio group such as methylthio, ethylthio, n-propylthio, isopropylthio, n-butylthio, isobutylthio, tert-butylthio and the like, among which methylthio, ethylthio, n-propylthio, n-butylthio, tert-butylthio or the like is preferable, and methylthio, ethylthio or n-butylthio is more preferable.

The "$C_1$-$C_4$ alkylamino" includes, for example, a linear or branched alkylamino group such as methylamino, ethylamino, n-propylamino, isopropylamino, n-butylamino, isobutylamino, tert-butylamino and the like, among which methylamino, ethylamino, n-propylamino, n-butylamino, isobutylamino, tert-butylamino or the like is preferable, and methylamino, ethylamino or n-butylamino is more preferable.

The "N,N-di-$C_1$-$C_4$ alkylamino" includes, for example a di-linear or branched alkylamino group such as dimethylamino, diethylamino, di-n-propylamino, diisopropylamino, di-n-butylamino, diisobutylamino, di-tert-butylamino and the like, among which dimethylamino, diethylamino, di-n-propylamino, di-n-butylamino, diisobutylamino, di-tert-butylamino or the like is preferable, and dimethylamino, diethylamino or di-n-butylamino is more preferable.

The "$C_5$ -$C_{12}$ aryl" includes, for example, cyclopentadienyl, phenyl, indenyl, biphenylyl, naphthyl and the like, among which phenyl is preferable.

The "$C_4$-$C_{10}$ heterocycle optionally containing, as a hetero atom, one to four members selected the group consisting of oxygen, a group of the formula: $S(\rightarrow O)n^6$ ($n^6$ is 0, 1 or 2), a group of the formula: N→O, a group of the formula: =N- and a group of the formula: $NR^{36}$ ($R^{36}$ is $C_1$-$C_4$ alkyl or benzyl) " includes, for example, a heterocycle group such as furyl, thienyl, 1-benzylpyrrolyl, 1-benzylimidazolyl, quinolyl, isoquinolyl, pyridyl, indolyl, pyrimidinyl, pyrazinyl and the like, among which furyl, thienyl, 1-benzylpyrrolyl, 1-benzylimidazolyl, quinolyl, isoquinolyl or pyridyl is preferable.

The "$C_7$-$C_{13}$ aralkyl" includes, for example, an aralkyl group such as benzyl, phenethyl, indenylmethyl, biphenylylmethyl, naphthylmethyl and the like, among which benzyl or naphthylmethyl is preferable.

The "$C_1$-$C_4$ alkylsulfonyl" includes, for example, a linear or branched alkylsulfonyl group such as methylsulfonyl, ethylsulfonyl, n-propylsulfonyl, isopropylsulfonyl, n-butylsulfonyl, isobutylsulfonyl, tert-butylsulfonyl and the like, among which methylsulfonyl, ethylsulfonyl, n-propylsulfonyl, n-butylsulfonyl, tert-butylsulfonyl or the like is preferable, and methylsulfonyl, ethylsulfonyl or n-butylsulfonyl is more preferable.

The "$C_2$-$C_5$ alkanoyl" includes, for example, a linear or branched alkanoyl group such as acetyl, n-propionyl, n-butyryl, isobutyryl, valeryl, pivaloyl and the like, among which acetyl, n-butyryl, isobutyryl, pivaloyl or the like is preferable, and acetyl, n-butyryl or pivaloyl is more preferable.

The "$C_2$-$C_5$ alkoxycarbonyl" includes, for example, a linear or branched alkoxycarbonyl group such as methoxycarbonyl, ethoxycarbonyl, n-propoxycarbonyl, isopropoxycarbonyl, n-butoxycarbonyl, isobutoxycarbonyl, tert-butoxycarbonyl and the like, among which ethoxycarbonyl, n-propoxycarbonyl, n-butoxycarbonyl, tert-butoxycarbonyl or the like is preferable, and ethoxycarbonyl, n-butoxycarbonyl or tert-butoxycarbonyl is more preferable.

The "$C_3$-$C_{12}$ alkylene" includes, for example, a linear alkylene group such as trimethylene, tetramethylene, pentamethylene, hexamethylene, heptamethylene, octamethylene, nonamethylene, decamethylene, undecamethylene, dodecamethylene and the like, among which trimethylene, tetramethylene, pentamethylene, nonamethylene, decamethylene, undecamethylene, dodecamethylene or the like is preferable, and trimethylene, tetramethylene, decamethylene, undecamethylene or dodecamethylene is more preferable.

The "$C_4$-$C_7$ alkylene" includes, for example, a linear alkylene group such as tetramethylene, pentamethylene, hexamethylene, heptamethylene and the like, among which tetramethylene, pentamethylene, hexamethylene or the like is preferable, and tetramethylene or pentamethylene is more preferable.

Next, the production method of the present invention will be explained below. According to the production method of the present invention, a halogen atom of the halide which has a group containing an acidic proton and in which one or more halogen atoms is/are substituted with the carbon atom of the carbon-carbon double bond is exchanged with a metal atom, and subsequently an electrophilic reagent is introduced to the carbon atom to which said metal atom is attached. As a result of introduction of an electrophilic reagent, a lactone ring may be formed in the case where a carboxyl group is present and a hydroxy group is formed in the product.

The method of the present invention is carried out by reacting a halide, which has a group containing an acidic proton and in which one or more halogen atom(s) is/are substituted on a carbon atom of the carbon-carbon double bond, with a magnesium halide represented by the formula (I), a magnesium compound represented by the formula (II), or a mixture thereof, followed by successive reactions with a lithium compound represented by the formula (III), and then with an electrophilic reagent, and optionally subjecting the resulting product to an acid treatment. The above reactions can be carried out in an industrially advantageous manner by preferably conducting the below-mentioned steps (1) to (3) continuously in numerical order.

Step (1) : A halide represented by the formula (IV) , which has a group containing an acidic proton and in which one or more halogen atoms is/are substituted with the carbon atom of the carbon-carbon double bond (hereinafter referred to as halide) is dissolved in an inert solvent, and a magnesium halide represented by the formula (I), a magnesium compound represented by the formula (II) , or a mixture thereof, is added to the halide at about -20°C to about 0°C over a period of about 0.5 to about 1 hour, and the reaction is carried out at about -30°C to about 40°C, preferably about -20°C to about 30°C, for about 0.5 to about 10 hours, preferably for about 0.5 to about 5 hours.

Step (2): A lithium compound represented by the formula (III) is added to the solution obtained in the step (1) at about -20°C to about 0°C over a period of about 0.5 to about 1 hour, and the reaction is carried out at about -30°C to about 40°C, preferably about -20°C to about 30°C, for about 0.5 to about 5 hours, preferably for about 0.5 to about 2 hours.

Step (3): An electrophilic reagent is added to the solution obtained in the step (2) at about -20°C to about 0°C over a period of about 0.5 to about 1 hour, and the reaction is carried out at about -20°C to about 40°C, preferably at about -20°C to about 30°C, for about 0.5 to about 5 hours, preferably about 0.5 to about 2 hours.

Examples of the inert solvent used in the steps (1) to (3) are preferably those which are inert and can dissolve the halide and any starting materials used in the halogen-metal exchange reaction such as the above-mentioned magnesium compound and lithium compound. Such a solvent includes, for example, preferably tetrahydrofuran, dioxane, dimethoxyethane, diethyl ether, tert-butyl methyl ether, benzene, toluene, n-hexane, n-heptane, and the like all of which do not participate in the reaction.

The amount of the magnesium halide represented by the formula (I) or a mixture thereof to be used is about 0.5 to about 3 equivalents, preferably about 0.5 to about 2.0 equivalents, to the halide. Further, two or three compounds selected from magnesium compounds represented by the formula (I) may be appropriately used in combination.

The amount of the magnesium compound represented by the formula (II) or a mixture thereof to be used is about 0.5 to about 2 equivalents, preferably about 0.5 to about 1.5 equivalents, to the halide. In addition, two or three compounds selected from magnesium compounds represented by the formula (II) may be appropriately used in combination.

The amount of a mixture of magnesium halides represented by the formula (I) and magnesium compounds represented by the formula (II) is about 0.5 to about 2 equivalents, preferably about 1 to about 1.5 equivalents, to the halide. Further, two or three compounds selected from the group consisting of magnesium halides represented by the formula (I) and magnesium compounds represented by the formula (II) may be appropriately used in combination.

The amount of the lithium compound represented by the formula (III) is about 1 to about 3 equivalents, preferably about 1 to about 1.5 equivalents, to the halide.

Examples of the lithium compound represented by the formula (III) include methyllithium, n-butyllithium, sec-butyllithium, tert-butyllithium, phenyllithium and the like, among which n-butyllithium is preferable.

The amount of the electrophilic reagent to be used is about 1 to about 2 equivalents to the halide.

If desired, a step of acid treatment may be carried out. The acid treatment is conducted, if desired, in the case where the reaction product obtained in the step (3) forms a lactone ring or the reaction product is extracted with an organic solvent. The specific condition for the step is, for example, that the step is carried out using an aqueous solution of about 1 to about 20 equivalents of ammonium chloride, formic acid, trifluoroacetic acid, citric acid or acetic acid, or hydrochloric acid or a dilute hydrochloric acid to the chloride at about 0°C to about 50°C for about 1 to about 10 hours.

The product obtained in the above step can be purified and separated by per se conventional methods such as column chromatography using silica gel, adsorbent resin, etc., liquid chromatography, solvent extraction or recrystallization/reprecipitation, solely or in combination, if required.

The starting halide used in the method of the present invention can be prepared by the conventional methods or an analogous method thereof, and known compounds or commercially available products, if any, may also be utilized.

Further, when the desired product in which a substituent is introduced in accordance with the method of the present invention contains, for example, a carboxyl group, an acidic group of the formula: $-SO_3H$ or a basic group such as amino groups, the desired product may be prepared in the form of a free acid, a free base, or a salt with a base (e.g. ammonia, dimethylamine, etc.) or a salt with an acid (e.g. hydrochloric acid, etc.).

## EXAMPLES

The invention is further specifically described below according to Examples, but the invention is not limited thereby at all.

Example 1

Preparation of 1'-benzylspiro[isobenzofuran-1(3H),4'-piperidin]-3-one hydrochloride

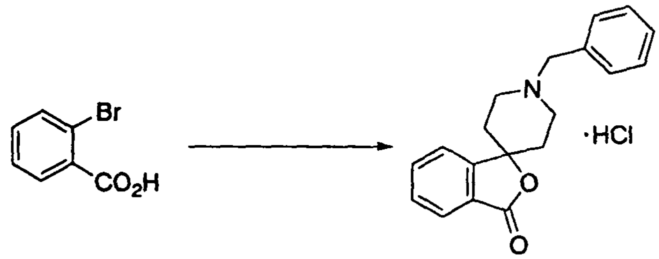

A solution of 2-bromobenzoic acid (10 g, 49.7 mmol) in tetrahydrofuran (100 ml) was cooled to -15°C in an ice-bath under nitrogen atmosphere, and to this solution was dropwise added 2.0M n-butylmagnesium chloride/tetrahydrofuran solution (22.4 mL, 44.8 mmol) at -5°C or lower. Then, 1.56M n-butyllithium/n-hexane solution (41.5 mL, 64.7 mmol) was dropwise added thereto at -10°C or lower over a period of 30 minutes, and the mixture was stirred at -15°C to -10°C for 1 hour, followed by dropwise addition of 1-benzyl-4-piperidone (10.1 mL, 54.5 mmol) in n-heptane (30 mL) at -15°C to -10°C over a period of 1 hour. After stirring at -15°C to -10°C for 1 hour, the water-bath was removed, and tert-butyl methyl ether (50 mL) was added, followed by addition of an aqueous solution (100 mL) of acetic acid (15.7 mL, 274 mmol) . The mixture was stirred at 35°C to 40°C for 3 hours, during which time white insoluble materials were dissolved completely. The reaction solution was cooled to room temperature and then separated into an organic layer and an aqueous layer. The aqueous layer was extracted with tert-butyl methyl ether (50 mL), and the organic layers were combined. To the extract were successively added 2M aqueous sodium hydroxide (50 mL, 100 mmol) and aqueous potassium carbonate (5 g, 36.2 mmol, 25 mL), and the mixture was stirred, then separated into an aqueous layer and an organic layer. The organic layer was washed twice with water (50 mL) and concentrated in vacuo to a volume of 60 mL under heating at 40°C, and methanol (120 mL) was added thereto. This procedure was repeated three times to replace the solution with the methanol solution while removing the water azeotropically (water content: 0.16% as estimated by Karl Fisher method) . The methanol solution was concentrated in vacuo to a volume of 61 mL under heating at 40°C, and 4M HCl/ethyl acetate solution (11.6 mL, 46.4 mmol) was dropwise added over a period of 30 minutes, whereby the precipitated crystalline free amine was dissolved and then the hydrochloride salt was immediately precipitated as colorless crystals. After stirring at room temperature for 30 minutes under nitrogen atmosphere, tert-butyl methyl ether (122 mL) was added slowly over a period of 1 hour. The mixture was stirred overnight at room temperature to give crystals. The crystals were collected by filtration under nitrogen atmosphere, washed twice with a mixture of methanol and tert-butyl methyl ether (1:5, 37 mL), and dried overnight in a desiccator at room temperature to yield the title compound (11.30 g, yield 69 %) as colorless crystals.
M.p. 275°C
IR(KBr) ν $cm^{-1}$:3430,2940,2540,1760,1470,1270,1070,930,740, 690
$^1$H-NMR(DMSO-$d_6$) δ ppm:1.94(2H,d,J=14Hz),2.83(2H,t,J=13Hz), 3.1-3.5(4H,m),4.45(1.8H,d,J=5Hz),4.76(0.2H,d, J=5Hz),7.4-7. 55(4H,m),7.6-7.75(3H,m),7.75-7.9(2H,m),11.5-11.8(1H,brs)

Example 2

Preparation of 1'-benzylspiro[isobenzofuran-1(3H),4'-piperidin]-3-one hydrochloride

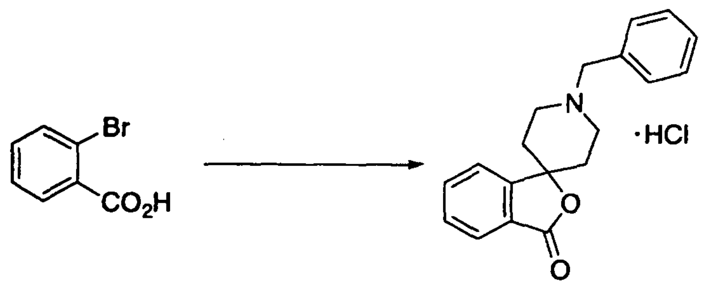

A solution of 2-bromobenzoic acid (100 g, 497 mmol) in tetrahydrofuran (1000 mL) was cooled to -20°C under nitrogen atmosphere, and to this solution was dropwise added 1.0M dibutylmagnesium/heptane solution (259 mL, 259 mmol; available from Aldrich) at -5°C or lower. After dropwise and gradual addition of 1. 56M n-butyllithium/n-hexane solution (351 mL, 548 mmol) at -15°C or lower over a period of 1 hour, the mixture was stirred at -17°C to -15°C for 1 hour, and a solution of 1-benzyl-4-piperidone (100 mL, 539 mmol) in tetrahydrofuran and n-heptane solution (1:6, 350 mL) was dropwise added at -17°C to -15°C over a period of 90 minutes. The mixture was stirred at -17°C to -15°C for 1 hour and the water-bath was removed. After addition of tert-butyl methyl ether (500 mL) followed by addition of aqueous solution (1 L) of acetic acid (114 mL, 1.99 mol), the mixture was stirred at 35°C to 40°C for 3 hours, during which time white insoluble materials were dissolved completely. The reaction solution was cooled to room temperature and tert-butyl methyl ether (500mL) was added thereto. The mixture was stirred and then separated into an organic layer and an aqueous layer. The aqueous layer was extracted with tert-butyl methyl ether (500 mL), and the extract was combined with the organic layer. The combined extracts were washed once with aqueous solution (750 mL) of potassium carbonate (150 g, 1.09 mol) and twice with water (500 mL). The organic layer was concentrated in vacuo to a volume of 472 mL under gentle heating at 40°C, and then methanol (945 mL) was added thereto. This procedure was repeated three times to replace the solution with the methanol solution while removing the water azeotropically (water content: 746 ppm as estimated by Karl Fisher method). The methanol solution was concentrated in vacuo to 472 mL under gentle heating at 40°C, and 4M HC1/ethyl acetate solution (100 mL, 400 mmol) was dropwise added over a period of 30 minutes, thereby the precipitated crystals of the free amine were dissolved, and then the hydrochloride salt was immediately precipitated as colorless crystals. After stirring at room temperature for 30 minutes under nitrogen atmosphere, tert-butyl methyl ether (1417 mL) was slowly added thereto over a period of 90 minutes. The mixture was stirred overnight at room temperature, and then the crystals were collected by filtration under nitrogen atmosphere, then washed successively with a mixed solution of methanol, and then tert-butyl methyl ether (1:5, 283 mL) and tert-butyl methyl ether (283 mL) . The crystals were dried overnight at room temperature in a desiccator to give colorless crystals. Since spectrometric analysis data (IR spectra and NMR spectra) of the resulting colorless crystals was consistent with those of the compound of Example 1, said colorless crystals (94.74 g, yield 58 %) were identified as the compound of Example 1.

Example 3

Preparation of 1-phenylisobenzofuran-3-one

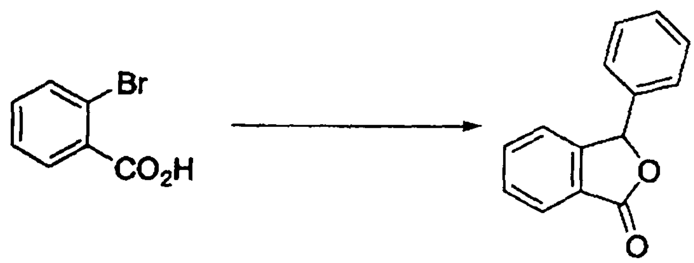

A solution of 2-bromobenzoic acid (1 g, 4.97 mmol) in tetrahydrofuran (10 mL) was cooled to -15°C under nitrogen atmosphere, and to this solution was dropwise added 1.0M dibutylmagnesium/heptane solution (2.6 mL, 2.6 mmol; available from Aldrich) at -5°C or lower, followed by dropwise and gradual addition of 1.56M n-butyllithium/n-hexane solution (3.4 mL, 5.30 mmol) at -15°C or lower over a period of 20 minutes. The mixture was stirred at -15°C for 1 hour, and then a solution of benzaldehyde (1.0 mL, 9.84 mmol) in n-heptane (3 mL) was dropwise added at -15°C or lower. The mixture was stirred at -15°C or lower for 1 hour, and 2M HCl (10 mL) was added thereto. The mixture was stirred overnight at room temperature, and ethyl acetate (20 mL) was added thereto. The mixture was stirred and then separated into an organic layer and an aqueous layer. The organic layer was washed successively with water (5 mL), 5 % aqueous sodium bicarbonate (10 mL), water (5 mL) and saturated brine (5 mL). The organic layer was dried over anhydrous sodium sulfate, filtered and concentrated in vacuo. The resulting solid was purified by column chromatography on silica gel (Wako gel C-300, eluent: ethyl acetate/n-heptane) to yield the title compound (919 mg, yield 88 %) as colorless crystals.
M.p. 118°C
IR(KBr)ν $cm^{-1}$:3060,3030,1750,1610,1600,1460,1290,1210,1070, 970,740
$^1$H-NMR($CDCl_3$) δ ppm:6.41(1H,s),7.2-7.5(6H,m),7.55(1H,t,J= 7.5Hz),7.65(1H,t,J=7.5Hz),7.97(1H,d,J=7.5Hz)

Examples 4 to 7

Compounds of Examples 4 to 7 were prepared in an analogous manner to Example 3.

Example 4

Preparation of 3-hydroxy-3H-isobenzofuran-1-one

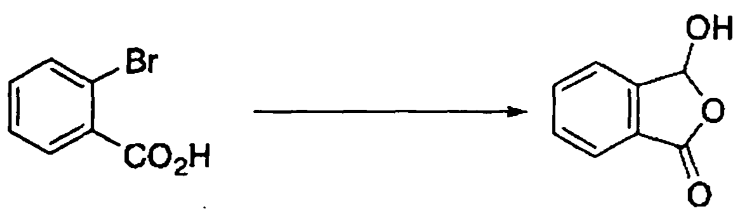

Following an analogous method to that described in Example 3 and using 2-bromobenzoic acid (1 g) and N, N-dimethylformamide (0.76 mL), there was prepared 3-hydroxy-3H-isobenzofuran-1- one (536 mg, yield 72 %).
$^1$H-NMR ($CDCl_3$) δ ppm:4.58(1H,brs),6.82(1H,brs),7.4-7.7 (3H,m), 7.86(1H,d,J=7.6Hz)

Example 5

Preparation of 3-n-hexyl-3H-isobenzofuran-1-one

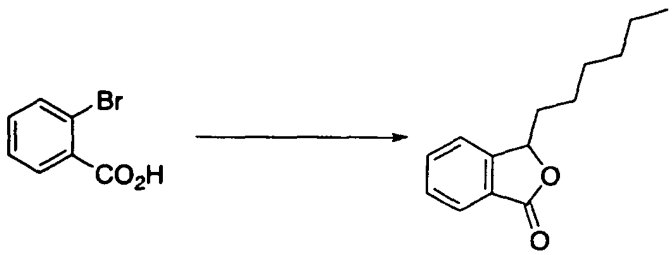

Following an analogous method to that described in Example 3 and using 2-bromobenzoic acid (1 g) and n-heptanal(0.88 mL), there was prepared 3-n-hexyl-3H-isobenzofuran-1-one (966 mg, yield 89 %).
$^1$H-NMR ($CDCl_3$) δ ppm: 1.2-1.55 (11H,m) , 1.75 (1H,m), 2.03 (1H,m), 5.48(1H,dd,J=8Hz,4Hz),7.44(1H,d,J=7.6Hz), 7.52(1H,t,J=7.6Hz ),7.67(1H,t,J=7.6Hz),7.90(1H,d,J=7.6Hz)

Example 6

Preparation of 3-ethyl-3-methyl-3H-isobenzofuran-1-one

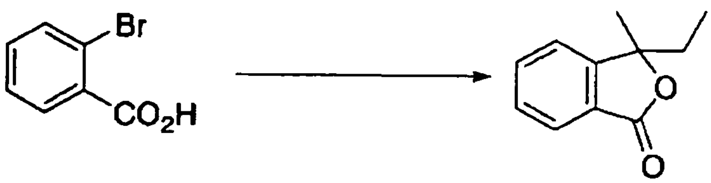

Following an analogous method to that described in Example 3 and using 2-bromobenzoic acid (1 g) and methyl ethyl ketone (0.54 mL), there was prepared 3-ethyl-3-methyl-3H-isobenzofuran-1-one (567 mg, yield 65 %).
$^1$H-NMR ($CDCl_3$) δ ppm:0.76(3H,t,J=7.3Hz),1.64(3H,s),1.93 (1H,m), 2.07 (1H,m),7.36 (1H,d,J=7.6Hz),7.51 (1H,t, J=7.6Hz), 7.67(1H,t,J=7.6Hz),7.87(1H,d,J=7.6Hz)

Example 7

Preparation of spiro[cyclohexane-1,1',(3'H)-isobenzofuran]-3'-one

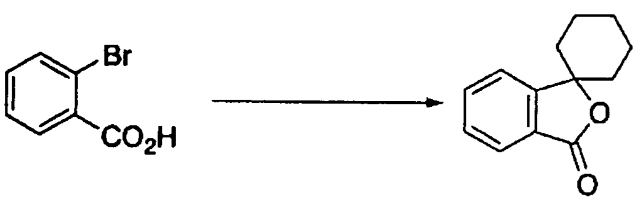

Following an analogous method to that described in Example 3 and using 2-bromobenzoic acid (1 g) and cyclohexanone (0.62 mL), there was prepared spiro[cyclohexane-1,1',(3'H)-isobenzofuran]-3'-one (606 mg, yield 60 %).
$^1$H-NMR ($CDCl_3$ ) δ ppm:1.6-2.0 (10H,m),7.40 (1H,d,J=7.6Hz),7.50 (1H,t,J=7.6Hz),7.65 (1H,t,J=7.6Hz),7.87 (1H,d,J=7.6Hz)

Example 8

Preparation of 3-(1'-hydroxybenzyl)benzoic acid

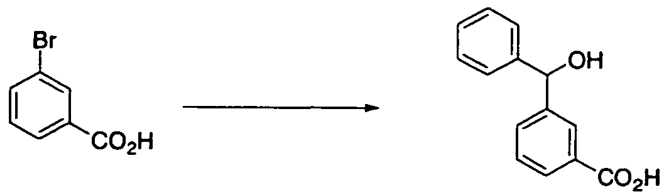

A solution of 3-bromobenzoic acid (1 g, 4.97 mmol) in tetrahydrofuran (10 mL) was cooled to -15°C under nitrogen atmosphere, and to this solution was dropwise added 1.0M dibutylmagnesium/heptane solution (2.6 mL, 2.6 mmol; available from Aldrich) at -10°C or lower. After dropwise and gradual addition of 1.56M n-butyllithium/n-hexane solution (3.5 mL, 5.30 mmol) at -15°C or lower over a period of 20 minutes, the mixture was stirred at -15°C for 1 hour, and then a solution of benzaldehyde (0.76 mL, 7.48 mmol) in n-heptane (3 mL) was dropwise added at -10°C or lower. The mixture was stirred at -15°C or lower for 1 hour and 2M HCl (10 mL) was added thereto. The mixed solution was stirred at room temperature for 30 minutes, and ethyl acetate (20 mL) was added thereto. The mixture was stirred and then separated into an organic layer and an aqueous layer. The organic layer was washed successively with water (5 mL) and saturated brine (5 mL), dried over anhydrous sodium sulfate, filtered, and concentrated in vacuo to remove the solvent. The resulting solid was purified by column chromatography on silica gel (Wako gel C-300, eluent: ethyl acetate/n-heptane) to yield the title compound (775 mg, yield 68 %) as colorless crystals.
$^1$H-NMR (DMSO-$d_6$) δ ppm:5.78(1H,s),6.02(1H,brs),7.2-7.5 (6H,m). 7.63(1H,d,J=8Hz),7.79 (1H,t,J=7.5Hz),7.97(1H,s)

Examples 9 to 13

Following an analogous method to that described in Example 8, compounds of Examples 9 to 13 were prepared.

Example 9

Preparation of 4-(1'-hydroxybenzyl)benzoic acid

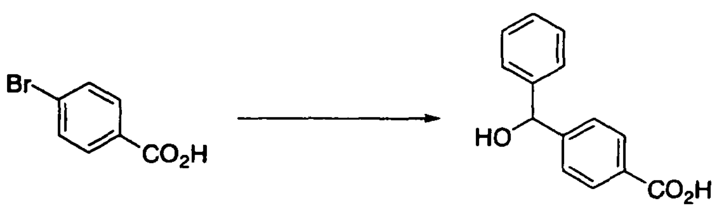

Following an analogous method to that described in Example 8 and using 4-bromobenzoic acid (1 g) and benzaldehyde (0.76 mL), there was prepared 4-(1'-hydroxybenzyl)benzoic acid (808 mg, yield 71 %).
$^1$H-NMR (DMSO-$d_6$) δ ppm:5.77(1H,s), 6.09(1H,brs), 7.2-7.5 (5H,m), 7.50(2H,d,J=8Hz),7.89(2H,d,J=8Hz)

Example 10

Preparation of 2-methylbenzoic acid

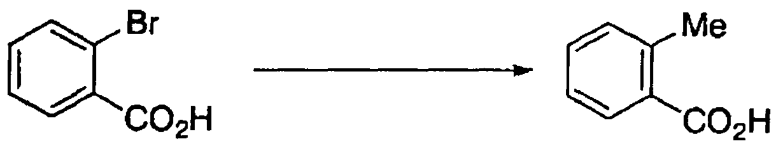

Following an analogous method to that described in Example 8 and using 2-bromobenzoic acid (1 g) and methyl iodide (0.62 mL), there was prepared 2-methylbenzoic acid (469 mg, yield 69 %).
$^1$H-NMR (DMSO-$d_6$) δ ppm:2.67(3H,s),7.2-7.35(2H,m),7.46(1H,m), 8.08(1H,d,J=8Hz)

Example 11

Preparation of 2-(1'-hydroxybenzyl)benzyl alcohol

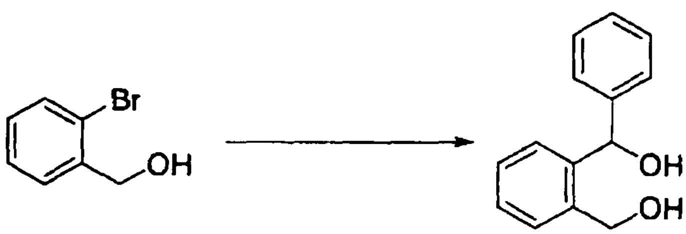

Following an analogous method to that described in Example 8 and using 2-bromobenzyl alcohol (930 mg) and benzaldehyde (1.0 mL), there was prepared 2-(1'-hydroxybenzyl)benzyl alcohol (943 mg, yield 89 %).
$^1$H-NMR($CDCl_3$) δ ppm:3.1(1H,brs),3.9(1H,brs),4.45(1H,d,J= 12Hz),4.61(1H,d,J=12Hz),6.01(1H,s),6.9-7.5(9H,m)

Example 12

Preparation of 2-(1'-hydroxybenzyl)-N-ethylbenzamide

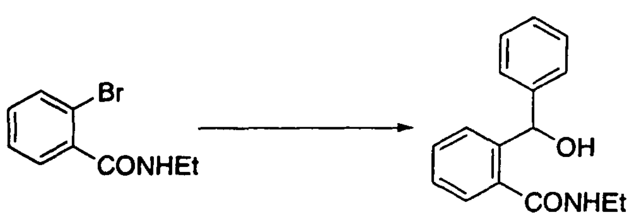

Following an analogous method to that described in Example 8 and using 2-bromo-N-ethylbenzamide (1.13 g) and benzaldehyde (0.76 mL), there was prepared 2-(1'-hydroxybenzyl)-N- ethylbenzamide (733 mg, yield 58 %).
$^1$H-NMR($CDCl_3$) δ ppm:0.76(3H,t,J=7.3Hz),3.1-3.3(2H,m),5.53 (1H,brs),5.87(1H,s),5.91(1H,brs),7.2-7.35(8H,m),7.40 (1H,d, J=7Hz)

Example 13

Preparation of 2-(1'-hydroxybenzyl)phenol

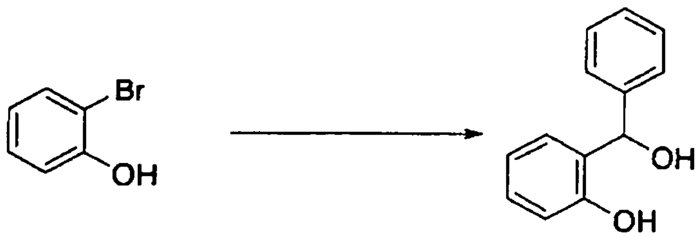

Following an analogous method to that described in Example 8 and using 2-bromophenol (0.58 mL ) and benzaldehyde (1.0 mL), there was prepared 2-(1'-hydroxybenzyl)phenol (913 mg, yield 91 %).
$^1$H-NMR ($CDCl_3$) δ ppm:3.07(1H,brs),5.99(1H,s),6.75-6.95 (3H,m), 7.1-7.5(6H,m),7.91(1H,s)

INDUSTRIAL APPLICABILITY

The method of introducing a substituent by halogen-metal exchange reaction in accordance with the present invention can reduce the production cost of compounds useful as pharmaceuticals, because, in the process of the present invention, there is no improper limitation in view of industrial production, such as steps of protection and deprotection after the reaction in the case where a starting halide contains a substituent of an acidic proton, which are required in the hitherto known methods, and the condition of extreme low temperature.

## Claims

**1.** A method of exchanging a halogen atom of a halide with a metal atom by halogen-metal exchange reaction and introducing an electrophilic reagent into a carbon atom to which the metal atom is attached, which comprises reacting a halide which has a group containing an acidic proton and in which one or more halogen atom(s) is/are substituted on a carbon atom of the carbon-carbon double bond, with a magnesium halide represented by the formula (I):

[I]

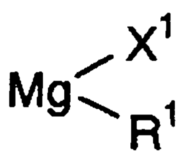

(wherein $R^1$ is a hydrocarbon group, and $X^1$ is a halogen atom), or a magnesium compound represented by the formula (II):

[II]

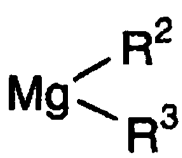

(wherein $R^2$ and $R^3$ are each independently a hydrocarbon group), or a mixture thereof, followed by successive reactions with a lithium compound represented by the formula (III):

[III]

$$Li-R^4$$

(wherein $R^4$ is a hydrocarbon group), and then with an electrophilic reagent, and then optionally treating the resulting product with an acid.

2. The method according to Claim 1, wherein the hydrocarbon group is an alkyl group, an aromatic group or an aralkyl group.

3. The method according to Claim 1 or 2, wherein the halide which has a group containing an acidic proton and in which one or more halogen atom(s) is/are substituted with the carbon atom of the carbon-carbon double bond is a compound represented by the formula (IV):

[IV]

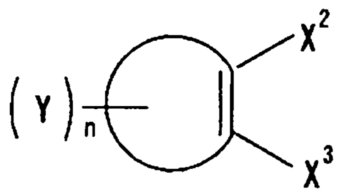

wherein

$X^2$ is a halogen atom;

$X^3$ is a hydrogen atom or a halogen atom;

n is an integer of 1 to 4;

Y is a carboxyl group, a hydroxymethyl group, a hydroxy group, a sulfamoyl group, a group of the formula: $-SO_3H$, a phosphono group, a group of the formula: $-NH-Y^1$ (wherein $Y^1$ is a hydrogen atom, a hydroxy group, an alkyl group, a carboxyl group, an aromatic group, a benzyloxycarbonyl group, an alkoxycarbonyl group or a phenyloxycarbonyl group), a group of the formula: $-CO-NH-Y^2$ ($Y^2$ is a hydrogen atom, an alkyl group or an aromatic group) or a group of the formula: $-NH-SO_2-Y^3$ (wherein $Y^3$ is a hydrogen atom, an alkyl group or an aromatic group), and

the group represented by the formula:

[V]

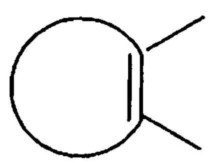

is a divalent homocyclic or heterocyclic group, and when n is more than 1, each Y may be the same or different.

4. The method according to Claim 2 or 3, wherein the alkyl group is a linear or branched $C_1$-$C_6$ alkyl group, and the aromatic group is phenyl, 1-naphthyl, 2-naphthyl or benzyl.

5. The method according to any one of Claims 1 to 4, wherein the homocyclic or heterocyclic ring is a benzene ring, a naphthalene ring, an indene ring, an indane ring, a pyridine ring, a pyridazine ring, a pyrimidine ring, a pyrazine ring, a thiophen ring, a furan ring, a pyran ring, an isobenzofuran ring, a pyrrole ring, an imidazole ring, a pyrazole

ring, an isothiazole ring, a thiazole ring, an oxazole ring, an isoxazole ring, a quinoline ring, an isoquinoline ring, a benzofuran ring, an indole ring, an isoindole ring, a phthalazine ring, a quinoxaline ring, a benzimidazole ring, a 1,8-naphthyridine ring, a benzoxazole ring, a benzothiazole ring, a benzothiophen ring, a cinnoline ring or a quinazoline ring.

**6.** The method according to any one of Claims 1 to 5, wherein the magnesium halide represented by the formula (I) is methyl magnesium bromide, methyl magnesium chloride, ethyl magnesium chloride, n-propyl magnesium chloride, isopropyl magnesium chloride, isopropyl magnesium bromide, n-butyl magnesium chloride, n-butyl magnesium bromide, isobutyl magnesium chloride, phenyl magnesium chloride or phenyl magnesium bromide, and the magnesium compound represented by the formula (II) is dimethylmagnesium, diethylmagnesium, di-n-propylmagnesium, diisopropylmagnesium, di-n-butylmagnesium, diisobutylmagnesium, di-sec-butylmagnesium, n-butyl(sec-butyl)magnesium or diphenylmagnesium.

**7.** The method according to any one of Claims 1 to 6, wherein the lithium compound represented by the formula (III) is methyllithium, n-butyllithium, sec-butyllithium, tert-butyllithium or phenyllithium.

**8.** The method according to any one of Claims 1 to 7, wherein the electrophilic reagent is benzaldehyde, methyl iodide, N,N-dimethylformamide, 1-benzyl-4-piperidone, 1-tert-butoxycarbonyl-4-piperidone, allyl bromide, cyclohexanone, n-heptanal, n-hexanal, acetone or methyl ethyl ketone.

**9.** The method according to Claim 1, which comprises reacting 2-bromobenzoic acid represented by the formula:

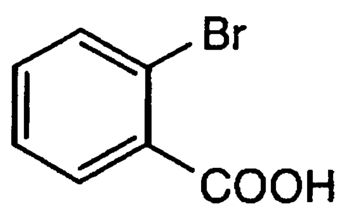

with di-n-butylmagnesium, di-sec-butylmagnesium, n-butyl(sec-butyl)magnesium, isopropyl magnesium chloride, n-butyl magnesium bromide, or a mixture thereof, followed by successive reactions with n-butyllithium, and then with a piperidone derivative represented by the formula:

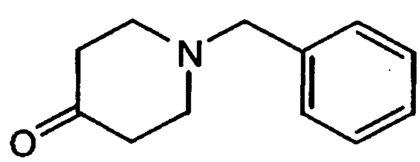

, and then treating the product with an acid to give a spiro compound represented by the formula:

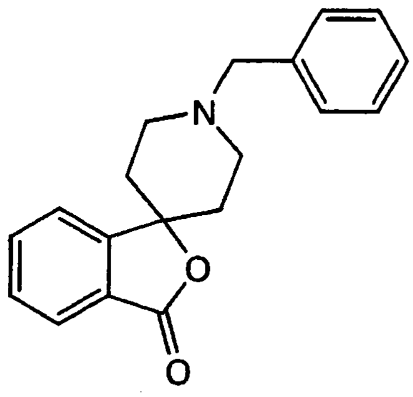

**10.** A method of the preparation of the spiro compound described in Claim 9, which comprises using the method according to Claim 9.

## INTERNATIONAL SEARCH REPORT

International application No.
PCT/JP03/06624

A. CLASSIFICATION OF SUBJECT MATTER
Int.Cl[7] C07B37/04, C07C51/367, 65/105, 51/353, 63/04, C07C29/40, 33/26, 231/12, 235/42, 37/20, 39/12, C07D491/107, 307/88, 307/89, 307/94

According to International Patent Classification (IPC) or to both national classification and IPC

B. FIELDS SEARCHED

Minimum documentation searched (classification system followed by classification symbols)
Int.Cl[7] C07B37/04, C07C51/367, 65/105, 51/353, 63/04, C07C29/40, 33/26, 231/12, 235/42, 37/20, 39/12, C07D491/107, 307/88, 307/89, 307/94

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | WO 01/57046 A1 (BANYU PHARMACEUTICAL CO., LTD.), 09 August, 2001 (09.08.01), & AU 2880501 A | 1-10 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 July, 2003 (07.07.03) | 29 July, 2003 (29.07.03) |
| Name and mailing address of the ISA/ Japanese Patent Office | Authorized officer |
| Facsimile No. | Telephone No. |

Form PCT/ISA/210 (second sheet) (July 1998)